# EUROPEAN PATENT APPLICATION

(11) **EP 2 368 529 A1**
(43) Date of publication of application: **28.09.2011**
(21) Application number: 11466004.6
(22) Date of filing: 02.03.2011
(51) Int. Cl.: A61F 2/44

(54) **Connecting intervertebral cage implant**

(30) Priority: 03.03.2010 CZ 20100155
(71) Applicant: Bertagnoli, Rudolf, 1190 Wien (AT)
(72) Inventor: Srámek, Jiri, 170 00 Praha 6 (CZ); Václav, Jirsák, 120 00 Praha 2 (CZ)
(74) Representative: Skoda, Milan

(57) **Abstract**

A stand-alone cage for vertebral interbody fusion, consisting of a corpus (1) that has at least one threaded opening (4) in which a threaded shaft (2,10) with teeth (5) is screwed. At least a further opening (6) in the corpus (1) with a worm shaft (3) in it. The thread (7) of the worm shaft (3) mate with the teeth (5) of the threaded shaft (2,10).

## Description

### Technical Field

The invention concerns a device to fuse vertebral bodies in spine surgery.

### The Existing State of the Art

Posterior Lumbar Interbody Fusion (PLIF) and Transforaminal Lumbar Interbody Fusion (TLIF) are surgical techniques to stabilize the lumbar spine involving surgical approach and the follow-up application of a bio-compatible material into the space of the intervertebral disc. The indication includes mostly degenerative affections of the lumbar spinal column, sometimes spondylolisthesis or even traumatic affections but very seldom. There are analogies with the aforementioned abbreviations, namely: ALIF (Anterior Lumbar Interbody Fusion) and ACIF (Anterior Cervical Interbody Fusion). There are no PCIF or TCIF techniques.

First, a posterior approach to the lumbar spine is made (either the standard approach in the centre line or the paramedial transmuscular one). Then, in case of using the PLIF technique, the flavum ligament between the discs of those vertebrae that are indicated for fusion is resected. The resection should be made in the minimum extent of the height of the intervertebral disc. Sometimes it shows necessary to resect part of the arch as well to make a better access. Then the dural sac is retracted medially, the posterior longitudinal ligament is exposed and the discectomy made. In case of using the TLIF technique introduced by Harms and Jeszensky, the approach into the intervertebral space is led more laterally via the intervertebral facet joint. Into the space created in this way, bone mash or a solid spongious bone graft worked into the desired shape or (at the present time prevailing) various implants are applied. The implants, mostly used at the present time, can be divided into to basic types - straight cages with the necessity of application of two implants into one intervertebral space and banana-shaped cages which rotate through an angle of 90 degrees to reach a symmetrical position in the intervertebral space. The advantage of these cages is the unilateral approach only which results in smaller postoperative epidural scarring. The cages can be divided into circular-shaped cages, mostly provided with a thread (these cages are to be screwed in the intervertebral space provided always that the cavity of the cage is filled with bone grafts), into straight ones with rectangular section and into those with the base the contour of which is banana-shaped.

For better stability, the cages are, in most cases, combined with posterior stabilization either transpedicular or translaminar. If the cage is placed separately, i.e. without any supplementary stabilization, the technique is called "stand-alone".
As the stand-alone technique bears the considerable risk of no or insufficient healing and of formation of a non-union, it is used relatively rarely. For that reason, implants that should (using various mechanisms) ensure the desired stability without the supplementary posterior fixation (expandable PLIF cages) have been developed. Expandable PLIF cages are those cages which change their size using a threaded wedge that slides inside the cage. The better stability of these cages is, however, only illusory because in all the cages the stability is ensured by the weight (pressure) of the body above the cage and the expansibility of the cage (the change of height of the cage in the intervertebral space) does not change this parameter. Another direction of development of the PLIF (TLIF) stand-alone cages is a supplementary mechanism which, after being introduced, gets into contact with the cover plates and bodies of the adjacent vertebrae. They are, for example, the cages with a keel (or fin) which however can injure nerve roots or the implants where, after moving the mechanism at the end of the cage round a slight amount, two triangles in contact with the cover plate are formed. All the aforementioned implants are straight PLIF (TLIF) cages which have therefore to be introduced in pairs.

### The Nature of the Invention

The aforementioned weaknesses are, to a large extent, eliminated and the goals of the invention accomplished by a stand-alone cage, especially a stand-alone cage for vertebral interbody fusion containing a corpus according to this invention, the principle of which lies in the fact that the corpus includes at least one threaded opening in which a threaded shaft with teeth is screwed provided always that there is at least another opening in the corpus in which a worm shaft is placed the thread of which mates with the teeth on the threaded shaft.

To improve penetration into the vertebral bodies, it is advantageous if the threaded shaft is provided with at least one self-cutting element.

Moreover, it is advantageous if the worm shaft is, on at least one of its ends, provided with a hole for the assembly instrument, preferably if the hole is in the shape of the Phillips drive or if it can accommodate a hexagonal or torx inner wrench.

It is important to prevent the worm shaft and the corpus from mutual disconnection when introduced. For that reason, it is recommendable that the worm shaft is provided with grooves at its ends which facilitate its rotation inside the corpus and, at the same time, prevent the worm shaft and the corpus from disconnection.

It is advantageous if at least one pair of the threaded openings is arranged symmetrically in the corpus with threaded and toothed shafts screwed in each of them on the understanding that the corpus has another opening with a worm shaft the thread of which mates with the teeth on the threaded shafts.

It is the most advantageous if two pairs of the threaded openings are arranged symmetrically in the corpus with threaded shafts screwed in each of them on the understanding that there are another two openings between the threaded openings with a worm shaft the thread of which mates with the teeth on the threaded shafts. This arrangement is the most advantageous in view of stable fixation of the stand-alone cage in the vertebral bodies.

It is further advantageous if the threaded openings are provided with threads that are not in the same directions.

The threaded openings in which the threaded shafts are placed do not have to be perpendicular to the base of the corpus but to ensure the best possible penetration of the threaded shaft into the vertebral body, it is advantageous it the threaded opening is perpendicular to the base of the corpus.

In view of the optimum force transmission in the worm gearing, it is advantageous if the threaded opening (in which the threaded and toothed shaft is screwed) is perpendicular to the opening in which the worm shaft is placed.

It is also advantageous if the contour of the corpus base is banana-shaped with the openings for the worm shafts the axes of which intersect in front of the concave side of the corpus. This is important with unilateral approach. Optionally, the axes of the opening for the worm shafts can be arranged in parallel.

The corpus can also advantageously be in the shape of a rectangular parallelepiped. The surface of the corpus can be finished to improve its stabilization in the place of application and to support the toleration by the patient.

The teeth can be made on a part of the threaded shaft only which prevents the shaft from its full unscrewing.

It is further advantageous if the threaded shaft is hollow which improves its penetration into the vertebral bodies. The worm shaft can be hollow as well.

It is also advantageous if the diameter of the shaft of the threaded shaft is variable. This prevents the shaft from its full unscrewing.

The stand-alone cage for vertebral interbody fusion with the stability ensured by using threaded shafts is advantageously used for such fusion and is implantable through the posterior approach (both interlaminar and transforaminal) to the lumbar spine and the sacrum.

Unlike various implants that are used today, the stand-alone cage according to this invention facilitates stabilization of the given segment of the spinal column through the least invasive approach similar to that commonly used for microdiscectomy, i.e. through a very small surgical wound (2 to 4 cm) without any need for additional stabilization with transpedicular or other screws. The stand-alone cage further enables the operating surgeon to use the unilateral surgical approach thus combining decompression and stabilization of the spinal segment with the least invasive approach which results in the minimum surgical stress.

### Outline of Individual Figures on the Drawing

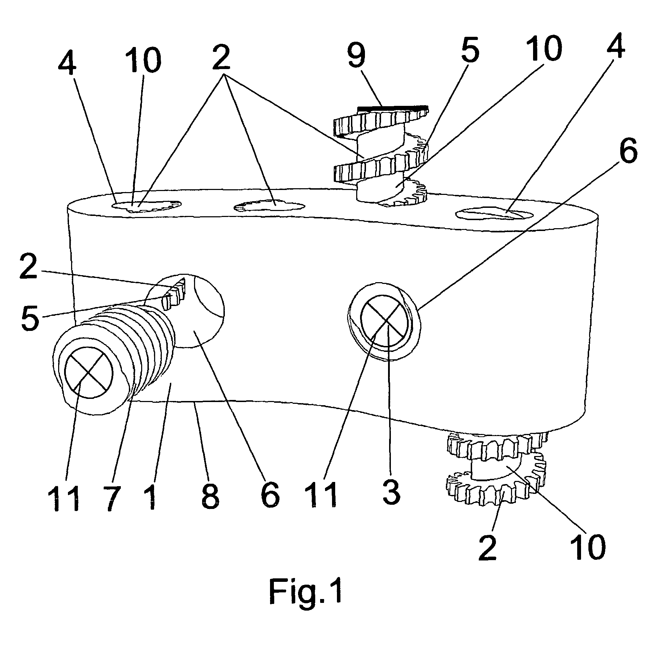

The nature of the invention is clarified in more detail on the drawing where Fig. 1 shows an axonometric view of the general arrangement of the stand-alone cage where two threaded shafts are positioned inside the corpus and two threaded shafts are partly outside the corpus in the position after implantation to stabilize the vertebrae.

### An Example of the Design of the Invention

The stand-alone cage (Fig. 1) for the vertebral body fusion consists of a corpus 1 in which two pairs of threaded openings 4 are symmetrically arranged. Inside the openings are screwed the threaded shafts 2 provided with teeth 5. Moreover, in the corpus 1, between the threaded openings 4 are two other openings 6 in which two worm shafts 3 are placed. The thread of the worm shafts 7 mates with the teeth on the threaded shafts 2. In this way, four worm gearings are created between the two worm shafts 3 and the teeth 5 made on the threads of the four threaded shafts 2.

The threaded shafts 2 are provided with a self-cutting element 9 (self-cutting blade) at the end.

On one of their ends, the worm shafts 3 are provided with the Phillips drive 11 for the assembly instrument.

Moreover, the worm shafts 3 are provided with grooves at their ends (not shown) which facilitate rotation of the worm shafts 3 inside the corpus 1 and, at the same time, prevent the worm shaft 3 and the corpus 1 from mutual disconnection.

The threaded openings 4 are provided with threads in the same direction and are arranged perpendicularly to the base 8 of the corpus 1.

The threaded openings 4, inside which the threaded shafts 2 with the teeth 5 are screwed, are arranged perpendicularly to the openings 6 with the worm shafts 3 inside.

The contour of the base 8 of the corpus 1 is banana-shaped and includes openings 6 for the worm shafts 3 the axes of which intersect in front of the concave side of the corpus 1. The worm shafts 3 pass through the concave side of the corpus 1 and are ended in front of the convex side of the corpus 1.

The sections of the threaded shafts 2 are solid but they can, optionally, be hollow.

The shafts 10 of the threaded shafts 2 have the same diameter along the whole of their length but the diameter can, optionally, be variable. The teeth 5 are made along the whole length of the threads 9 on the threaded shafts 2. Optionally, the teeth 5 can be made just along a part of the threads 9 on the threaded shafts 2. The sections of the worm shafts 3 are solid but they can, optionally, be hollow.

First, the stand-alone cage is inserted into the intervertebral space. Then, when turning the worm shafts 3, the threaded shafts 2 rotate and screw themselves off the corpus 1 into the vertebral bodies by which they stabilize the given segment of the spinal column.

In other advantageous designs or variants (not shown), the corpus can be in the shape of a rectangular parallelepiped and can be provided with one or more openings for threaded shafts and with one or more openings for worm shafts. The axes of the openings for the threaded shafts can be positioned symmetrically and do not have to be perpendicular to the base of the corpus. The axes of the worm shafts can be parallel. The threads of the threaded shafts do not have to be in the same direction.

### Industrial Utility

The stand-alone PLIF and TLIF cage for vertebral interbody fusion with the stability ensured by using threaded shafts is intended to be used in the field of spine surgery.

## Claims

1. The stand-alone cage, especially the stand-alone cage for vertebral interbody fusion, consisting of a corpus (1), **distinguishing itself** by the fact that at least one threaded opening (4) is arranged in the corpus (1) inside of which a threaded shaft (2) provided with teeth (5) is screwed on the understanding that there is at least another opening (6) in the corpus (1) with a worm shaft (3) the thread (7) of which mates with the teeth on the threaded shaft (2).

2. The stand-alone cage according to Claim 1 **distinguishing itself** by the fact that the threaded shaft (2) is provided with at least one self-cutting element (9) at its end.

3. The stand-alone cage according to some of the previous Claims **distinguishing itself** by the fact that the worm shaft (3) is provided with a drive for the respective assembly instrument minimally at one of its ends.

4. The stand-alone cage according to some of the previous Claims **distinguishing itself** by the fact that the worm shaft (3) is provided with grooves at its ends which facilitate its rotation inside the corpus (1) and, at the same time, prevent the worm shaft (3) and the corpus (1) from mutual disconnection.

5. The stand-alone cage according to some of the previous Claims **distinguishing itself** by the fact that at least one pair of threaded openings (4) is arranged inside the corpus (1) inside of which threaded shafts (2) with teeth (5) are screwed, provided always that there is another opening (6) in the corpus (1) with a worm shaft (3) in it the thread (7) of which mates with the teeth (2) on the threaded shafts.

6. The stand-alone cage according to some of the previous Claims **distinguishing itself** by the fact that two pairs of threaded openings (4) are arranged in the corpus (1) inside of which threaded shafts (2) with teeth (5) are screwed it being understood that between the threaded openings (4) in the corpus (1) are another two openings (6) with a worm shaft (3) in them the thread (7) of which mates with the teeth on the threaded shafts (2).

7. The stand-alone cage according to some of Claims 5 or 6 above **distinguishing itself** by the fact that the threaded openings (4) are provided with threads in the same direction.

8. The stand-alone cage according to some of the previous Claims **distinguishing itself** by the fact that the threaded opening (4) is perpendicular to the base (8) of the corpus (1).

9. The stand-alone cage according to some of the previous Claims **distinguishing itself** by the fact that the threaded opening (4) inside of which the threaded shaft (2) with teeth (5) is screwed is perpendicular to the opening (6) inside of which the worm shaft (3) is placed.

10. The stand-alone cage according to some of Claims 6, 7, 8 or 9 above **distinguishing itself** by the fact that the corpus (1) with the banana-shape contoured base (8) includes openings (6) the axes of which intersect in front of the concave side of the corpus (1).

11. The stand-alone cage according to some of the previous Claims **distinguishing itself** by the fact that the teeth (5) are made only on part of the thread (9) on the threaded shaft (2).

12. The stand-alone cage according to some of the previous Claims **distinguishing itself** by the fact that the shaft (10) of the threaded shaft (2) has variable diameter.

13. The stand-alone cage according to some of the previous Claims **distinguishing itself** by the fact that the threaded shaft (2) is hollow.

14. The stand-alone cage according to some of the previous Claims **distinguishing itself** by the fact that the worm shaft (3) is hollow.
